**Europäisches Patentamt**

(19) **European Patent Office**

**Office européen des brevets**

(11) Publication number : **0 436 353 B1**

(12) **EUROPEAN PATENT SPECIFICATION**

(45) Date of publication of patent specification :
16.06.93 Bulletin 93/24

(51) Int. CI.⁵ : **A61M 16/04**

(21) Application number : **90313979.8**

(22) Date of filing : **20.12.90**

(54) Apparatus for the aerosol administration of medication.

(30) Priority : **03.01.90 US 460315**

(43) Date of publication of application :
**10.07.91 Bulletin 91/28**

(45) Publication of the grant of the patent :
**16.06.93 Bulletin 93/24**

(84) Designated Contracting States :
**CH DE FR GB LI**

(56) References cited :
**EP-A- 0 245 607**
**WO-A-89/05670**
**US-A- 4 270 530**
**US-A- 4 327 721**
**US-A- 4 669 463**

(73) Proprietor : **MINNESOTA MINING AND MANUFACTURING COMPANY**
**3M Center, P.O. Box 33427**
**St. Paul, Minnesota 55133-3427 (US)**

(72) Inventor : **Matson, Charles John, c/o Minnesota Mining and**
**Manufact. Co., 2501 Hudson Road, P.O. Box 33427**
**St. Paul, Minnesota 55133-3427 (US)**
Inventor : **Velasquez, David Joseph, c/o Minnesota Mining and**
**Manufact. Co., 2501 Hudson Road, P.O. Box 33427**
**St. Paul, Minnesota 55133-3427 (US)**

(74) Representative : **Baillie, Iain Cameron et al c/o Ladas & Parry, Altheimer Eck 2**
**W-8000 München 2 (DE)**

## Description

BACKGROUND OF THE INVENTION

This invention relates to an apparatus for the administration of medication, and in particular to the aerosol administration of medication through an endotracheal tube.

The primary goal of emergency therapy is to quickly and efficiently deliver the medication required during the crisis period. In many emergency situations the selection of the route of administration can be nearly as important as the selection of the medication. For example, while prompt intravenous administration of medication is usually the first choice, it is often not possible because of cardiovascular collapse which prevents the placement of either peripheral or central venous catheters.

The establishment of a patient airway, for example with an endotracheal tube, is usually the first action taken during emergency treatment. The endotracheal tube provides a route for the administration of injectable medication. By injecting medication in liquid form from a syringe into the central bore of an endotracheal tube, the medication is deposited on the epithelial surface of the upper airways and absorbed into the bronchial and pulmonary circulation. This method of administration of medication in liquid form has some disadvantages: some of the liquid medication is lost on the interior surface of the endotracheal tube; the liquid medication is distributed over only a relatively small Portion of the available pulmonary surfaces, delaying uptake and limiting the dosage that can be administered; it is time consuming to dilute the medication and load the syringe; and the use of this method of administration may require the interruption of CPR efforts.

Various endotracheal tubes have been constructed to facilitate the delivery of liquid medication through the endotracheal tube. For example the devices disclosed in US-A-4 739 756, 4 119 101, 4 669 463, and 4 821 714 each provide for the administration of medication in liquid form via an endotracheal tube. Furthermore WO-A- 8 905 670 discloses an endotracheal cannula which comprises a flexible internal tube for guiding atomized product into the bronchial free, wherein the free end opening of the tube has a diameter equal to that of its own lumen. However, these devices still suffer from the same disadvantages associated with the administration of medication in liquid form discussed above.

SUMMARY OF THE INVENTION

The apparatus of the present invention may be used in a method which involves administering medication in aerosol form through an endotracheal tube, and therefore alleviates some of the disadvantages encountered with the administration of liquid medication through an endotracheal tube. The aerosol administration of medication reduces the amount of medication lost on the interior surfaces of the endotracheal tube, and it permits the medication to be distributed over a larger surface area, speeding uptake and permitting larger dosages to be administered. The aerosol medication can be provided in pre-loaded canisters, since dilution is not critical, thereby saving the time required for diluting and loading syringes with liquid medication. Finally, the aerosol administration of medication need not interfere with on-going CPR efforts.

The apparatus of the present invention may be used in a method wherein medication is administered by inserting an endotracheal tube into the patient's trachea, and injecting medication in aerosol form into the air stream in the endotracheal tube. The medication is preferably injected at the center of the endotracheal tube in order to maximize its entrainment in the air stream and to minimize the deposition of the medication on the sidewalls of the tube.

The apparatus of this invention, which is defined in claim 1, is adapted for injecting medication in aerosol form into the air stream in an endotracheal tube. Generally, the apparatus comprises an improved endotracheal tube of the type comprising a hollow tube body having a proximal end, and a distal end adapted for insertion into the patient's trachea. The apparatus further comprises an aerosol medication lumen, having a proximal end and a distal end, and running at least partly along the endotracheal tube. An aerosol nozzle is disposed at the distal end of the medication lumen, inside the tube but adjacent its distal end. A connector is provided on the proximal end of the medication lumen for connecting to an aerosol canister of medication to provide the medication to the medication lumen for ejection in aerosol form from the nozzle.

Thus, the apparatus of this invention provides for the injection of medication in aerosol form into the air stream in an endotracheal tube, providing for better distribution of the medication over the pulmonary surfaces than can be achieved with the administration of liquid medication. This better distribution speeds uptake of the medication, and allows larger doses to be administered. Because the medication is in aerosol, rather than liquid form, less medication is lost on the surfaces of the endotracheal tube. The aerosol administration permits the use of metered dose canisters that eliminate mixing and measuring delays experienced with liquid medications. Finally, aerosol administration does not interfere with on-going CPR efforts.

These and other advantages will be in part apparent, and in part pointed out hereinafter.

BRIEF DESCRIPTION OF THE DRAWINGS

Figure 1 is a side elevation view of an improved endotracheal tube constructed according to the principles of this invention;

Figure 2 is an enlarged transverse cross-sectional view of the improved endotracheal tube taken along the plane of line 2-2 in Figure 1;

Figure 3 is a longitudinal cross-sectional view of the improved endotracheal tube taken along the plane of line 3-3 in Figure 2; and

Figure 4 is a side elevation view of a second embodiment of the improved endotracheal tube constructed according to the principles of this invention.

Corresponding reference numerals indicate corresponding parts throughout the several view of the drawings.

DETAILED DESCRIPTION OF THE PREFERRED EMBODIMENT

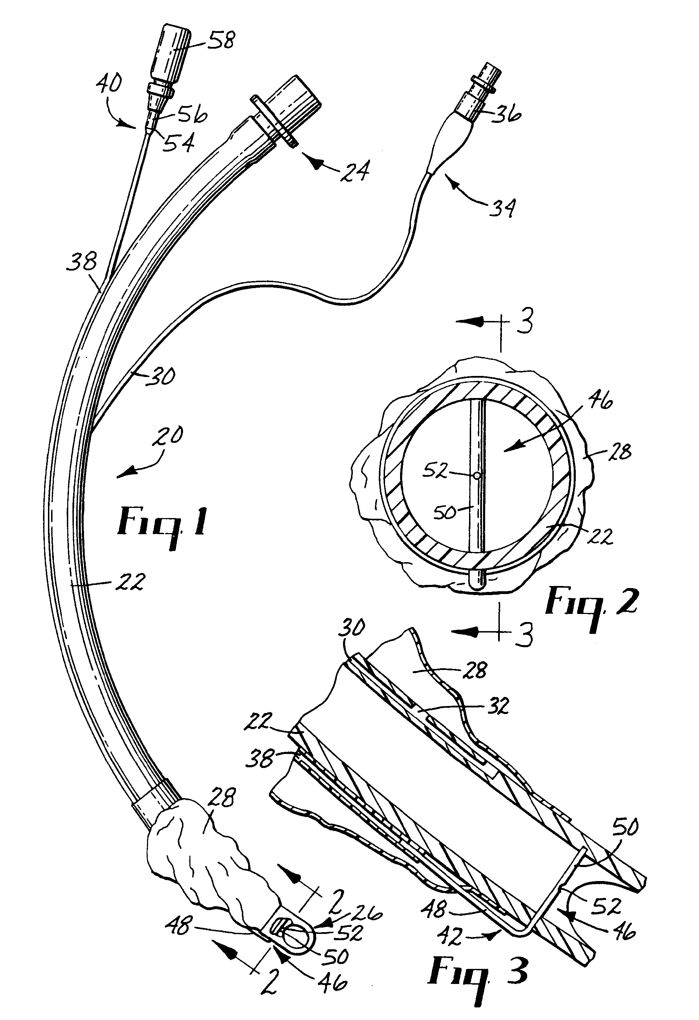

A first embodiment of an improved endotracheal tube constructed according to the principles of this invention is indicated generally as 20 in Figure 1. The endotracheal tube 20 is generally of standard construction, comprising a hollow tubular body 22, preferably made of a flexible plastic material. The body 22 has a proximal end 24, and a distal end 26 that is adapted for insertion into the trachea of a patient. For this purpose, the distal end 26 has a rounded tapering configuration. As is well known in the art, the endotracheal tube 20 may include an inflatable balloon 28 on its exterior, adjacent its distal end, and a lumen 30 extending substantially along the length of the endotracheal tube. The distal end 32 of the lumen 30 communicates with the balloon 28, and the proximal end 34 of the lumen 30 has a connector 36, adapted for connection to an air supply to inflate the balloon 28.

However, unlike a conventional endotracheal tube, the endotracheal tube 20 has an aerosol medication lumen 38, having a proximal end 40 and a distal end 42. The medication lumen 38 is preferably made from a flexible chemically-inert (i.e., inert to the medications to be delivered) polymeric material so that it can flex with the endotracheal tube, although it could be made of some other suitable material. The medication lumen 38 extends along at least a portion of the tubular body 22, and may be located inside the body, outside the body, or inside the wall of the body. The distal end 42 of the medication lumen 38 preferably terminates in an injector 46. The injector 46 comprises a first portion 48 extending generally parallel with the tubular body 22, and a second portion 50 extending generally chordally (and more preferably diametrically) across the interior of the tubular body 22, closely adjacent the distal end of the tubular body. The injector 46 is preferably a small diameter, L-shaped hollow metal tube. A nozzle 52 is preferably located in the middle of the second portion 50 of the injector 46, at the center (as considered in transverse cross-section) of the tubular body. The nozzle 52 is preferably oriented to face outwardly from the distal end of the endotracheal tube 20.

There is preferably some means, such as connector 54 for connecting the proximal end 40 of the medication lumen 38 to the valve and stem 56 of a canister 58 of aerosol medication, so that actuation of the canister 58 releases the medication into the medication lumen 38. The canister 58 is preferably of the type that can dispense metered doses upon actuation. These canisters typically contain multiple doses of a medication and are equipped with a metering valve to regulate the dosage. These valves are designed to deliver a predetermined volume of the aerosol formulation, e.g., 50 or 63 microliters, each time the valve is actuated. The lumen 38 conducts the medication under pressure to the nozzle 52, from which it is ejected as an aerosol into the air stream passing through the endotracheal tube 20. The generally central location of the nozzle 52 and its location adjacent the distal end of the endotracheal tube 22 causes most of the medication to be entrained in the air stream, with relatively little of the medication being deposited on the walls of the endotracheal tube.

The length and internal size of the lumen 38, the length and internal size of the injector 46, and the size of the nozzle 52 are all adjusted to achieve the best aerosol dispersion of the medication. Experiments indicate that greater efficiencies in delivery are achieved with larger dose sizes. Experiments have also indicated that better aerosol delivery is seen at smaller nozzle diameter (e.g., 5 μ) or larger nozzle diameter (e.g., 81 or 100 μ) but not at intermediate sizes. The appropriate orifice diameter, lumen diameter and lumen length for a given dosage are easily determined by testing. The following table tabulates the results of testing conducted by the inventors:

Remote Nozzle Geometries and Aerosol Outputs

| Orifice diameter (microns) | Lumen diameter (mm) | Lumen length (mm) | Aerosol output:** Respirable mass (mg/m3) |
|---|---|---|---|
| 100 | 2.92 | 152.4 | 2.6,3.5,3.4,2.8 |
| 81 | 2.92 | 152.4 | 0.3,0.4,0.3,0.9 |
| 50 | 1.57 | 152.4 | 0.1,0.1,0.1,2.5 |
| 33 | 1.19 | 152.4 | 0.1,0.3,1.3,59.2 |
| 23 | 1.14 | 152.4 | 0.1,0.1,0.3,7.8 |
| 13 | 1.14 | 152.4 | 0.0,0.0,0.0,34.1 |
| 5 | 1.14 | 152.4 | 0.1,0.1,0.1,27.0 |
| 100 | 2.92 | 304.8 | 3.3,4.4,3.2,4.0 |
| 81 | 2.92 | 304.8 | 1.1,1.0,0.7,1.0 |
| 50 | 1.57 | 304.8 | 0.1,0.1,0.0,0.0 |
| 33 | 1.19 | 304.8 | 0.0,0.0,0.0,0.1 |
| 23 | 1.14 | 304.8 | 0.0,0.0,0.2,3.3 |
| 13 | 1.14 | 304.8 | 0.0,0.0,0.0,0.0 |
| 5 | 1.14 | 304.8 | 0.2,0.1,0.0,31.0 |

**Aerosol output is reported as four values indicating output from four different MDI valve volumes: 25 μl, 50 μl, 63 μl, and 100 μl.

According to the present invention, the endotracheal tube body 22 is first inserted into the trachea of the patient to establish an air supply to the patient's lungs. Then, when medication is needed, connector 54 is connected to the valve and stem 56 of a canister 58 of the medication. The valve and stem 56 are actuated, and medication is delivered to the medication lumen 38, where it passes under pressure to the injector 46. The medication is ejected in aerosol form from the nozzle 52, and is entrained into the air stream in the endotracheal tube, and distributed throughout the lungs of the patient. Because of its aerosol form, the medication is distributed over a large surface area, and is rapidly taken up.

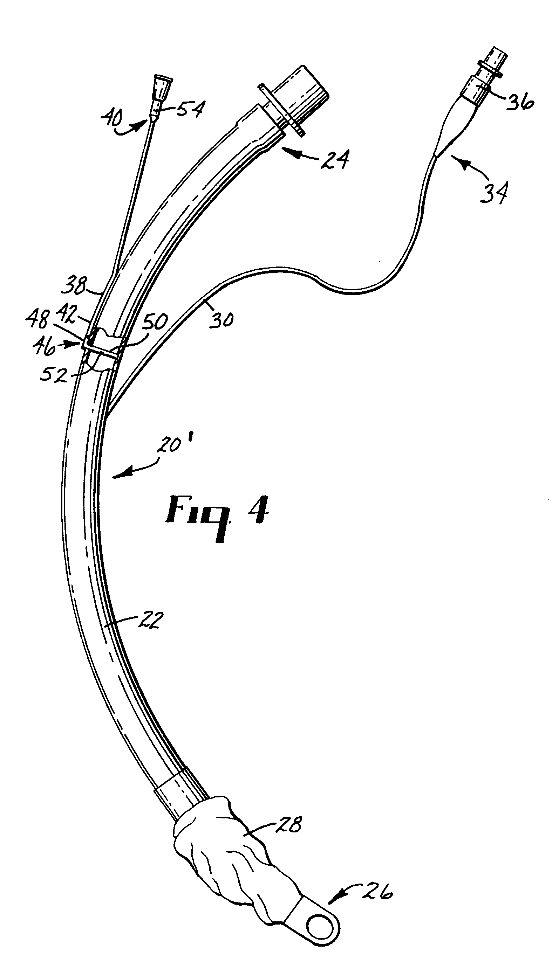

A second embodiment of an improved endotracheal tube constructed according to the principles of this invention is indicated generally as 20' in Figure 4. The endotracheal tube 20' is similar in construction to endotracheal tube 20, and corresponding parts are identified with corresponding reference numerals. The chief difference between tubes 20' and 20, is that in tube 20' the injector 46 and nozzle 52 are not located adjacent the distal end 26.

In view of the above, it will be seen that the several objects of the invention are achieved and other advantageous results attained.

As various changes could be made in the above constructions without departing from the scope of the invention, it is intended that all matter contained in the above description or shown in the accompanying drawings shall be interpreted as illustrative and not in a limiting sense.

4

## Claims

1. An endotracheal tube (20, 20') of the type comprising a hollow tubular body (22) having a proximal end (24), and a distal end (26) adapted for insertion into the trachea of a patient, said endotracheal tube (20, 20') comprising :
an aerosol medication lumen (38), having proximal (40) and distal ends (42), and extending along at least a portion of the tubular body; the distal end (42) of the lumen (38), being disposed inside the tubular body (22); and
means for connecting the proximal end (40) of the lumen (38) to a supply of aerosol medication (58), further characterized in that a nozzle (52) is provided at the distal end of the medication lumen, in order to dispense the medication in aerosol form from the nozzle (52) in the endotracheal tube (20, 20').

2. The endotracheal tube according to claim 1 wherein the nozzle (52) is located adjacent the distal end (26) of the tubular body (22).

3. The endotracheal tube according to claim 2 further comprising an injector (46) at the distal end (42) of the lumen (38), the injector (46) having a portion extending generally transversely across the tubular body (22), adjacent the distal end (26) of the tubular body, and wherein the nozzle (52) is located in the injector (46).

4. The endotracheal tube according to claim 1 or 2 further comprising an injector (46), the injector having a portion extending generally transversely across the tubular body (22), and wherein the nozzle is located in the injector (46).

5. The endotracheal tube according to claim 3 or 4 wherein the nozzle (52) is located in the injector (46) at a position generally at the center of the tubular body (22).

6. The endotracheal tube according to claim 3 or 4 wherein the nozzle (52) is oriented to face generally outwardly toward the distal end (26) of the tubular body (22).


## Patentansprüche

1. Endotrachealtubus (20, 20') der Art umfassend einen hohlen rohrförmigen Körper (22) mit einem proximalen Ende (24) und einem distalen Ende (26), das in die Luftröhre eines Patienten eingeführt werden kann, wobei der Endotrachealtubus (20, 20') folgendes umfaßt:
ein Aerosolverabreichungslumen (38) mit einem proximalen Ende (40) und einem distalen Ende (42), das sich entlang von mindestens einem Abschnitt des rohrförmigen Körpers erstreckt, wobei das distale Ende (42) des Lumens (38) innerhalb des rohrförmigen Körpers (22) angeordnet ist; und
eine Einrichtung, die das proximale Ende (40) des Lumens (38) an einen Behälter (58) für ein Medikament in Form eines Aerosols anschließt, ferner dadurch gekennzeichnet, daß eine Düse (52) am distalen Ende des Verabreichungslumens angeordnet ist, damit das Medikament in Form eines Aerosols aus der Düse (52) in den Endotrachealtubus (20, 20') abgegeben wird.

2. Endotrachealtubus nach Anspruch 1, dadurch gekennzeichnet, daß die Düse (52) im Bereich des distalen Endes (26) des rohrförmigen Körpers (22) liegt.

3. Endotrachealtubus nach Anspruch 2, ferner umfassend eine Einspritzvorrichtung (46) am distalen Ende (42) des Lumens (38), wobei die Einspritzvorrichtung (46) einen Abschnitt besitzt, der sich im Bereich des distalen Endes (26) des rohrförmigen Körpers im allgemeinen quer über den rohrförmigen Körper (22) erstreckt, und wobei die Düse (52) in der Einspritzvorrichtung (46) angeordnet ist.

4. Endotrachealtubus nach Anspruch 1 oder 2, ferner umfassend eine Einspritzvorrichtung (46), wobei die Einspritzvorrichtung einen Abschnitt aufweist, der sich im allgemeinen quer über den rohrförmigen Körper (22) erstreckt, und wobei die Düse in der Einspritzvorrichtung (46) angeordnet ist.

5. Endotrachealtubus nach Anspruch 3 oder 4, dadurch gekennzeichnet, daß die Düse (52) in der Einspritzvorrichtung (46) in einer Position im allgemeinen in der Mitte des rohrförmigen Körpers (22) angeordnet ist.

**6.** Endotrachealtubus nach Anspruch 3 oder 4, dadurch gekennzeichnet, daß die Düse (52) so ausgerichtet ist, daß sie im allgemeinen nach außen zum distalen Ende (26) des rohrförmigen Körpers (22) weist.

**Revendications**

**1.** Tube endotrachéal (20,20') du type comprenant un corps tubulaire creux (22) ayant une extrémité proche (24) et une extrémité distante (26) destinée à être insérée dans la trachée d'un patient, le dit tube endotrachéal (20,20') comprenant :

un conduit (38) de médicament pour aérosol, ayant une extrémité proche (40) et une extrémité distante (42) et s'étendant le long d'au moins une partie du corps tubulaire, l'extrémité distante (42) du conduit (38) étant située à l'intérieur du corps tubulaire (22) ; et

des moyens de raccordement de l'extrémité proche (40) du conduit (38) à une source de médicament pour aérosol (58) ;

caractérisé en outre en ce qu'une buse (52) est prévue à l'extrémité distante du conduit de médicament, afin de distribuer le médicament sous forme d'aérosol par la buse (52) dans le tube endotrachéal (20,20').

**2.** Tube endotrachéal suivant la revendication 1, dans lequel la buse (52) est située près de l'extrémité distante (26) du corps tubulaire (22).

**3.** Tube endotrachéal suivant la revendication 2, comprenant en outre un injecteur (46) à l'extrémité distante (42) du conduit (38), l'injecteur (46) comprenant une partie disposée sensiblement transversalement au corps tubulaire (22), près de l'extrémité distante (26) du corps tubulaire, et la buse (52) étant placée dans l'injecteur (46).

**4.** Tube endotrachéal suivant la revendication 1 ou 2, comprenant en outre un injecteur (46), l'injecteur comportant une partie qui s'étend sensiblement transversalement au corps tubulaire (22), et la buse étant située dans l'injecteur (46).

**5.** Tube endotrachéal suivant la revendication 3 ou 4, dans lequel la buse(52)est située dans l'injecteur (46) à une position sensiblement au centre du corps tubulaire (22).

**6.** Tube endotrachéal suivant la revendication 3 ou 4, dans lequel la buse (52) est tournée sensiblement vers l'extérieur, vers l'extrémité distante ou libre (26) du corps tubulaire (22).

*Fig. 1*

*Fig. 2*

*Fig. 3*

Fig. 4